# EUROPEAN PATENT APPLICATION

(11) **EP 3 770 602 A1**
(43) Date of publication of application: **27.01.2021**
(21) Application number: 19382627.8
(22) Date of filing: 23.07.2019
(51) Int. Cl.: G01N 33/569

(54) **IN VITRO METHOD FOR DISCRIMINATING LATENT FROM ACTIVE TUBERCULOSIS**

(71) Applicant: Fundació Assistencial de Mútua de Terrassa, FPC, 08221 Terrassa, Barcelona (ES); Universitat de Barcelona, 08028 Barcelona (ES); Fundació Institut D'investigació En Ciències De La Salut Germans Trias i Pujol, 08916 Badalona (ES)
(72) Inventor: PÉREZ PORCUNA, Tomás María, 08197 San Cugat ( Barcelona) (ES); COMELLA DEL BARRIO, Patricia, 08916 Badalona (Barcelona) (ES); DOMINGUEZ BENITEZ, Jose Antonio, 08916 Badalona (Barcelona) (ES); ABELLANA SANGRÁ, Rosa Mari, 08036 Barcelona (ES)
(74) Representative: Hoffmann Eitle

(57) **Abstract**

*In vitro* method for discriminating latent from active TB. The present invention refers to an *in vitro* method for the differential diagnosis between active TB, preferably early stages of TB, and LTBI.

## Description

### FIELD OF THE INVENTION

The present invention pertains to the medical field. Particularly, it refers to an *in vitro* method for the differential diagnosis between active TB (TB), preferably early stages of TB, and latent tuberculosis infection (LTBI).

### STATE OF THE ART

At least one-quarter of the world's population is infected with *Mycobacterium tuberculosis.* Childhood TB represents at least 10% of the burden of the disease worldwide, being one of the worlds significant causes of childhood morbidity and mortality. Estimations show that there are far more children with TB globally than previously thought, with the majority undiagnosed and untreated. Underdiagnosis in children can occur for reasons such as the lack of non-specific clinical presentation that delays seeking of healthcare and diagnostic suspicion, the lack of a more accurate diagnostic test, and the poor geographical and financial access to health care in some areas.

Infants and young children have a higher risk of progressing to TB following primary *M. tuberculosis* infection, usually due to a child exposed to an infectious adult with active TB, and to develop severe forms of the disease. Therefore, the household detection of TB index cases, together with early detection of the infection and TB disease, followed by a prompt treatment are fundamental to prevent disease progression.

TB diagnosis is a significant challenge in children due to the paucibacillary nature of the disease and the difficulties to expectorate. Although rapid molecular diagnosis represents a significant advance as an alternative to conventional microscopy and culture methods, its sensitivity remains unacceptably low in children since the technique is based on detecting *M. tuberculosis* from respiratory specimens. As for the immunodiagnostics assays, both tuberculin-skin-test (TST) and interferon-gamma release assays (IGRAs), that measure cell-mediated immune responses following *M. tuberculosis* infection, are unable to distinguish between active TB and LTBI and have limitations on the accuracy of *M. tuberculosis* detection. As a result, most of the active TB cases are diagnosed through clinical, and radiological when possible, scoring systems with limitations due to the clinical presentation of the disease. Moreover, primary TB in initial phase in children has a difficult diagnosis, because they mainly have only mediastinal manifestation (i.e. an early stage TB without pulmonary affectation) and lower clinical symptomatology. In addition, the X-ray, microbiology and molecular tests have low sensibility due to little bacilar load in this disease phase.

The immature immune system of young children added to the dynamic process of the host-pathogen interaction from *M. tuberculosis* infection, hamper to define the transition from latent infection to acute TB disease in children. Recently, a diversity of genetic and individual factors that may contribute to the outcome of the host immune set points during *M. tuberculosis* infection has been shown. A good understanding of the diversity of these factors might be crucial to assess the impact of the host-pathogen interactions that occur during *M. tuberculosis* infection. In this sense, WHO guidelines for the management of malnourished children, as well as guidelines for national TB programs, underline the importance of the association of malnutrition and TB in children. Besides, impacts of the alteration of iron homeostasis and vitamin D deficiencies highlight profound effects on immune function and host defenses due to mechanisms that have not yet been resolved. Lastly, comorbidities by helminth infections impair the inflammatory and immune mechanisms involved in the control of *M. tuberculosis* infection.

In recent years, pediatric research has focused on finding new biomarkers in non-sputum-based samples for the detection of *M. tuberculosis* infection. Current studies have highlighted the importance of diverse forms of biomarkers with the potential to be used in immunological methods for the diagnosis of childhood TB. Furthermore, increasing data has been published showing that modified IGRAs based on the analysis of a combination of different markers could enhance the accuracy of the diagnosis. However, only a few studies with small cohorts have identified some cytokines responses in QuantiFERON-TB Gold In-Tube (QFT-GIT, QIAGEN, Germany) supernatants with the potential to monitor specific immunity against *M. tuberculosis* as candidate combinations of markers for the discrimination between LTBI and active TB in children. Despite these initial approaches, a clear pattern has not yet emerged, and the diagnostic performance of cytokine biomarkers reported do not meet the minimum targets recommended by WHO for a new diagnostic or triage test for TB in children.

So, in summary, there is a clear unmet medical need in relation with the differential diagnosis between TB, preferably early stages of TB, and LTBI. Image techniques of the lung currently used for the diagnosis of TB give rise to the identification of false negatives. By using image techniques, there could be patients wrongly classified as negative for TB when they are actually suffering from an early stage TB which is not identified by image techniques.

The present invention is aimed at solving the above cited problems by providing a minimally-invasive *in vitro* method for the differential diagnosis between TB, preferably early stages of TB, and LTBI. This method is not only able to differentiate TB from LTBI, but it is also able to discriminate early stages of TB from LTBI. As explained above, since this method is able to identify early stages of TB, the percentage of false negatives currently classified by using image techniques and/or techniques based on detection *M. tuberculosis* such as microbiological and molecular test, would be reduced. Moreover, the identification of patients suffering from early stages of TB would give to the clinician the possibility of recommending a treatment in an early stage of the disease, thus decreasing the morbimortality and increasing the effectivity of the treatment.

### DESCRIPTION OF THE INVENTION

### Brief description of the invention

The present invention refers to an *in vitro* method for the differential diagnosis between TB, preferably early stages of TB, and LTBI. Moreover, the method of the invention is also able to discriminate TB from healthy subjects. Consequently, by using the method of the invention, the clinician will have the possibility to recommend a treatment if it is finally determined that the patients are suffering from TB and a preventive treatment or no treatment if the patients are suffering from LTBI. No treatment would be recommended by the clinician if it is concluded that the patients are not suffering from TB or LTBI (i.e. healthy subjects).

Particularly, this method comprises i) the stimulation of blood samples obtained from the patient with a *M. tuberculosis* antigen (Ag-TB) and/or with a mitogen, and ii) the identification of specific cytokines (preferably IP-10 and IFN-γ) for example directly by ELISA or indirectly by counting the number of T cells secreting the cytokines. Moreover, in a preferred embodiment, the method of the invention further comprises the identification of an acute-phase reactant (for example ferritin) and/or a vitamin D analogue (for example 25-hydroxyvitaminD; 25(OH)D).

By way of example, the detection of the biomarkers can be carried out by using the following procedure:
**1.** Cytokine's (IP-10 and IFN-γ) detection can be done using the following tubes:
   a. Nil tube: This is the negative control tube, without stimulant, used to adjust the background.
   b. Ag-TB tube: This tube enables immediate exposure of viable blood lymphocytes to highly specific antigens of *M. tuberculosis* (for example ESAT-6 or CFP-10), producing a quantifiable immune response to aid in the diagnosis of TB infection. The difference between the level of the cytokines IP-10 and IFN-γ in nil tube with respect to level of the cytokines IP-10 and IFN-γ in Ag-TB tube is determined in order to conclude if the patient is infected.
   c. Mitogen tube: This tube is in principle used as a positive control tube. It may comprise for example phytohaemagglutinin (PHA) as the mitogen used to trigger T-lymphocyte cell division. Low response may indicate inability to generate cytokines. However, it is important to note that, according to the present invention, the mitogen tube is not only used as a positive control but it is also used for the stimulation of blood samples. IP-10 and IFN-γ are also measured in this tube once blood samples have been stimulated with the mitogen. According to the present invention, such as it will be explained below, there is a specific relation among the levels of IP-10 and IFN-γ determined in both TB antigen tube and mitogen tube which allows the differentiation among TB and LTBI patients.
**2.** The detection of the acute-phase reactant (for example ferritin) and/or a vitamin D analogue (for example 25-hydroxyvitaminD; 25(OH)D) is carried out in the biochemical tube by using immunoassay methods.

Such as it can be seen in **Table 1,** although the method for differentiating TB vs LTBI can be performed by determining the level of specific cytokines (preferably IP-10 and IFN-γ) after the stimulation of blood samples obtained from the patient with Ag-TB and/or with a mitogen, in a preferred embodiment, the combination of IP-10, IFN-γ, acute-phase reactant (for example ferritin) and/or a vitamin D analogue (for example 25-hydroxyvitaminD; 25(OH)D) achieved the best diagnostic performance to discriminate between active TB and LTBI. Preferably, the model is based on the combination of four biomarkers, namely: IP-10, IFN-γ, ferritin and 25-hydroxyvitaminD (25(OH)D). The combination of the four biomarkers achieves the best diagnostic performance to discriminate between TB and LTBI, giving rise to a ROC curve with an AUC of 0.955, and sensitivity and specificity of 93.2% and 90.0% respectively.

**Table 1. Percentages of classification (%) and area under the ROC curve of the study groups.**

| **Model** | **Signature: active TB* vs LTBI** | **Patient classification** | | **AUC** |
|---|---|---|---|---|
| | | **% TB** | **% LTBI** | |
| **1** | **Ag** (IFN-γ) | 100 | 0 | 0.59 |
| **2** | **Ag** (IFN-γ + IP-10) | 88.6 | 27.3 | 0.72 |
| **3** | **Ag** (IFN-γ + IP-10) + **Mit** (IFN-γ + IP-10) | 86.4 | 59.1 | 0.84 |
| **4** | **Ag** (IFN-γ + IP-10) + **Mit** (IFN-γ + IP-10)+ Ferritin | 90.9 | 70.0 | 0.91 |
| **5** | **Ag** (IFN-γ + IP-10) +**Mit** (IFN-γ + IP-10)+ 25(OH)D | 90.9 | 63.6 | 0.87 |
| **6** | **Ag** (IFN-γ + IP-10) + **Mit** (IFN-γ + IP-10)+Ferritin + 25(OH) D | 90.0 | 93.2 | 0.95 |

| | | | | |
|---|---|---|---|---|
| TB, tuberculosis; LTBI, latent TB infection; **Ag,** lymphocytes stimulated with Ag-TB (QFT). **Mit,** lymphocytes stimulated with mitogen (PHA). Ag, lymphocytes stimulated with M. tuberculosis antigens (QFT) adjusted by basal levels; Mit, lymphocytes stimulated with mitogen (PHA) adjusted by basal levels; 25(OH)D, 25-hydroxyvitamin D.* In this table, patients with mediastinal TB were not included into the active TB cases since they are analysed separately in **Table 2.** | | | | |

Moreover, such as it can be seen in **Table 2,** although the method for differentiating early stages of TB vs LTBI can be performed by determining the level of specific cytokines (preferably IP-10 and IFN-γ) after the stimulation of blood samples obtained from the patient with Ag-TB and/or with a mitogen, in a preferred embodiment, the combination of IP-10, IFN-γ, acute-phase reactant (for example ferritin) and/or a vitamin D analogue (for example 25-hydroxyvitaminD; 25(OH)D) achieved the best diagnostic performance to discriminate between early stages of TB and LTBI. Preferably, the model is based on the combination of four biomarkers, namely: IP-10, IFN-γ, ferritin and 25-hydroxyvitaminD (25(OH)D). The combination of the four biomarkers achieves the best diagnostic performance to discriminate between early stages of TB and LTBI, giving rise to a ROC curve with an AUC of 0.83, and sensitivity and specificity of 93.2% and 76.5% respectively.

**Table 2. Percentages of classification (%) and area under the ROC curve of the study groups.**

| **Model** | **Signature: Mediastinal TB vs LTBI** | **Patient classification** | | **AUC** |
|---|---|---|---|---|
| | | **% Early stages of TB** | **% LTBI** | |
| **1** | **Ag** (IFN-γ) | 100% | 0.0% | 0.5 |
| **2** | **Ag** (IFN-γ + IP-10) | 88.9% | 27.3% | 0.58 |
| **3** | **Ag** (IFN-γ + IP-10) + **Mit** (IFN-γ + IP-10) | 83.3% | 59.1% | 0.71 |
| **4** | **Ag** (IFN-γ + IP-10) + **Mit** (IFN-γ + IP-10)+ Ferritin | 88.2% | 70.0% | 0.79 |
| **5** | **Ag** (IFN-γ + IP-10) + **Mit** (IFN-γ + IP-10)+ 25(OH)D | 88.9% | 63.6% | 0.76 |
| **6** | **Ag** (IFN-γ + IP-10) + **Mit** (IFN-γ + IP-10)+Ferritin + 25(OH) D | 76.5% | 93.2% | 0.83 |

| | | | | |
|---|---|---|---|---|
| TB, tuberculosis; LTBI, latent TB infection; **Ag,** lymphocytes stimulated with Ag-TB (QFT). **Mit,** lymphocytes stimulated with mitogen (PHA). Ag, lymphocytes stimulated with *M. tuberculosis* antigens (QFT) adjusted by basal levels; Mit, lymphocytes stimulated with mitogen (PHA) adjusted by basal levels; 25(OH)D, 25-hydroxyvitamin D. | | | | |

So, the methods described in the present invention comprise, as a first step, i) the stimulation of blood samples obtained from the patient with Ag-TB and/or with a mitogen and, as a second step, ii) the identification of specific cytokines (preferably IP-10 and IFN-γ) after performing the step i). Moreover, in a preferred embodiment, the method of the invention further comprises the identification of an acute-phase reactant (for example ferritin) and/or a vitamin D analogue (for example 25(OH)D).

Therefore, the first embodiment of the present invention refers to an *in vitro* method for the differential diagnosis between active TB and LTBI which comprises:
a) Determining the level of at least IFN-γ and IP-10 after the stimulation of blood samples obtained from the patient with Ag-TB;
b) Determining the level of at least IFN-γ and IP-10 after the stimulation of blood samples obtained from the patient with a mitogen;
c) Wherein if the level of IFN-γ after the stimulation with the mitogen is lower as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is an indication that the patient is suffering from active TB;
d) Wherein if the level of IFN-γ after the stimulation with a mitogen is higher as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is an indication that the patient is suffering from LTBI.

In a preferred embodiment, if the level of IFN-γ after the stimulation with the mitogen is lower as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is also an indication that the patient is suffering from early stage active TB.

In a preferred embodiment, the method further comprising the determination of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin, and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol, wherein if the level of the acute-phase reactant and/or the level of the vitamin D analogue is higher as compared with the level obtained in patients suffering from LTBI, this is an indication of active TB, preferably early stage active TB.

In a preferred embodiment, the mitogen is phytohaemagglutinin and the Ag-TB is ESAT-6 and/or CFP-10.

In a preferred embodiment, the patient is further analyzed by radiography (preferably of chest) and the diagnosis is confirmed by CT scan, mycobacterial culture, PCR of *M. tuberculosis* or by pathological anatomy.

The second embodiment of the present invention refers to the *in vitro* use of at least IFN-γ and IP-10, after the stimulation of blood samples obtained from the patient with Ag-TB and with a mitogen, for the differential diagnosis between active TB and LTBI, for the diagnosis of early stage active TB, for predicting the response to a treatment of patients suffering from active TB, for deciding whether to recommend a treatment to patients suffering from active TB or for deciding whether to recommend a preventive treatment to patients suffering from LTBI.

In a preferred embodiment, the present invention refers to the *in vitro* use of at least IFN-γ and IP-10, after the stimulation of blood samples obtained from the patient with Ag-TB and with a mitogen, for the diagnosis of early stage active TB.

In a preferred embodiment, said cytokines are used in combination with the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol.

The third embodiment of the present invention refers to a kit adapted for the differential diagnosis between active TB and LTBI, for the diagnosis of early stage active TB, for predicting the response to a treatment of patients suffering from active TB, for deciding whether to recommend a treatment to patients suffering from active TB or for deciding whether to recommend a preventive treatment to patients suffering from LTBI which comprises:
a) Reagents or media for the determination of the level of IFN-γ and IP-10 after the stimulation of blood samples obtained from the patient with Ag-TB, and
b) Reagents or media for the determination of the level of IFN-γ and IP-10 after the stimulation of blood samples obtained from the patient with a mitogen.

In a preferred embodiment, the kit further comprising reagents or media for the detection of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or for the detection of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol.

The fourth embodiment of the present invention refers to an *in vitro* method for predicting the response to a treatment of patients suffering from active TB which comprises:
a) Determining the level of at least IFN-γ and IP-10 after the stimulation of blood samples obtained from the patient with Ag-TB;
b) Determining the level of at least IFN-γ and IP-10 after the stimulation of blood samples obtained from the patient with a mitogen;
c) Wherein if the level of IFN-γ after the stimulation with a mitogen is higher as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is an indication that the patient is responding to the treatment.

In a preferred embodiment this method further comprising the determination of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol wherein if the level of the acute-phase reactant and/or vitamin D analogue is lower as compared with the level obtained in patients suffering from active TB, this is an indication that the patient is responding to the treatment.

The fifth embodiment of the present invention refers to an *in vitro* method for deciding whether to recommend a treatment to patients suffering from active TB which comprises:
a) Determining the level of at least IFN-γ and IP-10 after the stimulation of blood samples obtained from the patient with Ag-TB;
b) Determining the level of at least IFN-γ and IP-10 after the stimulation of blood samples obtained from the patient with a mitogen;
c) Wherein if the level of IFN-γ after the stimulation with the mitogen is lower as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, an anti-TB treatment is recommended.

The sixth embodiment of the present invention refers to an *in vitro* method for deciding whether to recommend a preventive treatment to patients suffering from LTBI which comprises:
a) Determining the level of at least IFN-γ and IP-10 after the stimulation of blood samples obtained from the patient with Ag-TB;
b) Determining the level of at least IFN-γ and IP-10 after the stimulation of blood samples obtained from the patient with a mitogen;
c) Wherein if the level of IFN-γ after the stimulation with a mitogen is higher as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, a preventive treatment is recommended.

In a preferred embodiment the method further comprises the determination of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol and/or falecalcitriol wherein if the level of the acute-phase reactant and/or vitamin D analogue is higher as compared with the level obtained in patients suffering from LTBI, an anti-TB treatment or a preventive treatment is recommended.

Thus the results provided in the present invention suggest the diagnostic potential of the combination of IFN-γ, IP-10, ferritin and/or 25(OH)D detected from supernatants in QFT-GIT tubes for the diagnosis of TB and the discrimination between TB, or early stages of TB, and LTBI. Besides, these markers could be useful for the identification of the onset of primary TB, guaranteeing future investigations in transversal and prospective longitudinal studies. Finally, developing a rapid diagnostic test based in the presented immunological biomarkers would improve the access of TB services, thus promoting the early diagnosis of TB and LTBI.

Such as it has been cited above, the determination of the level of IFN-γ and IP-10, in any of the methods described in the present invention, can be done by any means known in the prior art. By way of example, the determination of the cytokine levels can be carried out directly by ELISA, or indirectly by counting the number of T cells secreting said cytokines (spots), as a positive correlation exists between the number of responding cells and the amount of cytokine release [Diagnosing TB infection in children: analysis of discordances using in vitro tests and the tuberculin skin test. N. Altet-Gómez, et al., 2011.European Respiratory Journal 2011 37: 1166-1174; DOI: 10.1183/09031936.00022710]*.*

For the purpose of the present invention the following terms are defined:
- "Early stages of active TB" would include:
   ∘ Subclinical TB disease originated by viable *M. tuberculosis* bacteria that does not cause clinical TB-related symptoms but causes other abnormalities that can be detected using existing radiologic (such mediastinal affection) or microbiologic/molecular assays. Thus, in a preferred embodiment it is also included mediastinal manifestation (i.e. an early stage TB without pulmonary affectation).
   ∘ Incipient active TB disease originated by viable *M. tuberculosis* that causes minor clinical symptoms or radiographic abnormalities with or without microbiologic/molecular evidence/confirmation.
- In the present invention, different "reference values" are used in order to finally determined whether the patient is suffering from TB, preferably early stages TB, or LTBI. Precisely, a very specific correlation has been defined among the levels of IFN-γ and IP-10, once the blood cells have been stimulated with the antigen or the mitogen. Specifically, the methods described in the present invention use as "reference level" the level of IFN-γ obtained after the stimulation with the antigen and the level of IP-10 and IFN-γ obtained without stimulation, in order to determine if the patient is suffering from active TB. Moreover, the methods described in the present invention use as "reference level" the level of IFN-γ obtained after the stimulation with the antigen, and the level of IP-10 and IFN-γ obtained without stimulation, in order to determine if the patient is suffering from LTBI. Moreover, the present invention uses as "reference level" the level of the acute-phase reactant and/or the level of the vitamin D analogue in patients suffering from LTBI. The "reference level" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then, sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve.
- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of'. Thus, the phrase "consisting of" indicates that the listed elements are required or mandatory, and that no other elements may be present.

### Description of the figures:

**Figure 1****.** Flow diagram of enrolment. TB, tuberculosis; LTBI, LTBI; PTB, intrathoracic TB with pulmonary involvement; Mediastinal TB, intrathoracic TB with isolated mediastinal lymphadenopathy in the absence of lung parenchyma involvement.
**Figure 2****.** ROC curve of the four markers presented in the model [IFN-γ, IP-10, Ferritin, and 25(OH)D] to discriminate between active TB (N=44) and latent TB infected cases (N=37) with positive QuantiFERON-TB Gold In-Tube result. AUC, Area under ROC curve.

### DETAILED DESCRIPTION OF THE INVENTION

### Example 1. Materials and methods.

### 1.1 Study population

A prospective case-control study was conducted from August 2015 to December 2016 in the paediatric hospital of Saint Damien in Port-au-Prince (Haiti). Children (0-14 years old) who presented signs and symptoms compatible with active TB and/or documented TB exposure were screened for suspected TB. According to the hospital programme, siblings (0-14 years old) of the children diagnosed with TB were also screened for TB or LTBI. Children (0-14 years old) from a school and a kindergarten were screened as uninfected controls. The exclusion criteria were: children with known immunodeficiency, on current immunosuppressive treatment, with a condition that could potentially compromise the immune system (e.g. children from oncology, rheumatology, nephrology and those who had undergone organ transplantation), children who had been under anti-TB treatment or preventive treatment during the previous year, and children not providing informed consent.

The following information was collected: age, sex, weight, height, previous medical history (including TB history, TB exposure, HIV status, and comorbidities, haemogram), vaccines, and current and previous medication (antibiotics, corticosteroids, antiparasitic drugs).

The following signs and symptoms were evaluated: cough and/or fever ≥ two weeks (with no improvement after at least a seven-day course of amoxicillin), recent unexplained weight loss, and asthenia/fatigue. The TST was performed by a trained laboratory technician. Intradermal injection of 0.1 ml of Tubersol (bioequivalent to 5 tuberculin units; Sanofi Pasteur, Toronto, Canada) was placed into the ventral surface of the lower arm and read after 72 hours. A positive TST result was defined as an induration ≥ 10 mm in BCG-vaccinated children (those with a BCG scar), and ≥ 5 mm in non-BCG-vaccinated children or with a known adult TB contact (American Academy of Pediatrics, 2009; Bass et al., 1990). A standardised specific Z- score for detection of nutritional status weight-for-age (WAZ) was determined by WHO Anthro Plus 1.0.4 software (WHO AnthroPlus, 2009). Children with WAZ scores below -2 standard deviation (SD) were defined as underweight, and a WAZ score above 2 SD was defined as overweight.

A chest radiograph (anterior-posterior image) was performed in all the children screened for TB or LTBI. Chest radiographs were read by two external experts who were blinded to clinical data using a standardized reporting form and a third reader to resolve discordant opinions (Graham et al., 2015). In addition, nasopharyngeal aspirates and induced sputum were collected on three consecutive days for smear examination (auramine stain) by direct fluorescence microscopy in children suspected of having active TB. Histological examination was performed in children with suspicion of extrathoracic TB (ETB) with lymph node adenopathies.

Of all the children screened for TB or LTBI, only those with a positive TST result and/or microscopic confirmation were invited to participate in the study. Of all the children screened in the schools, only those with a negative TST result were invited to participate in the study as uninfected controls. Treatment and preventive treatment were prescribed in all the children diagnosed with TB or LTBI, respectively. During TB treatment and preventive treatment, the patients were followed monthly until the end of treatment. Children with a positive sputum smear were retested at the fifth and sixth month of treatment.

### 1.2 Ethics Statement.

This study was approved by the Health Research Ethics Committee of Barcelona University and the Haitian National Ethics Committee (project number IRB00003099). Before participation, written informed consent was obtained from the child's parent or guardian.

### 1.3 Definitions for classification of children enrolled in the study.

Children were classified according to their clinical history, chest radiographs, smear examination, molecular diagnostic test, TST and QFT-GIT results. Active TB cases were defined as confirmed TB -children with relevant signs and symptoms and microbiologic confirmation of *M. tuberculosis-,* and unconfirmed TB -children without bacteriological confirmation but with relevant signs and symptoms, positive TST and/or QFT-GIT, radiological findings suggestive of TB, known TB contact, and clinical response to anti-TB treatment. Depending on the TB location, active TB cases were classified as ETB or intrathoracic TB. Children with intrathoracic TB were differentiated into those with pulmonary involvement (PTB) and children with isolated mediastinal lymphadenopathy in the absence of lung parenchyma involvement (Mediastinal TB). LTBI cases were defined as children with documented TB exposure, positive TST and/or QFT-GIT, normal chest radiographs, and no clinical signs of TB development in the last six months after diagnosis. Finally, uninfected controls were defined as asymptomatic children with no history of TB exposure, and negative TST and QFT-GIT.

### 1.4 Laboratory tests performed.

### 1.4.1 Molecular diagnostic test.

Sputum samples were collected from children with microscopy confirmation and/or radiological findings to perform GeneXpert MTB/RIF (Cepheid, USA) according to the manufacturer's instructions.

### 1.4.2 QuantiFERON-TB Gold In-Tube.

In all the participants, a 3 ml blood sample was drawn for conventional QFT-GIT; the antigen tube (ESAT-6, CFP-10, and TB-7.7), and positive (phytohemagglutinin, mitogen) and negative (no antigen, nil) controls. Tubes were incubated at 37°C for 16 to 24 hours and centrifuged according to the manufacturer's instructions. Plasma was harvested and stored at -20°C until performing the enzyme-linked immunosorbent assay (ELISA). After the screening and recruitment of cases, plasma samples were sent to the laboratories of the Research Institute Germans Trias i Pujol (IGTP, Badalona, Spain) following optimum conservation conditions. Once there, the supernatants were analysed and the results were interpreted according to the manufacturer's instructions.

### 1.4.3 Ferritin, 25(OH)D, and Toxocara spp. detection.

One ml of blood from all the participants was aded to the biochemical tube to perform ferritin, 25(OH)D, and *Toxocara canis* determination. Ferritin and 25(OH)D concentrations were analysed at the biochemistry laboratory of the Germans Trias i Pujol Hospital (Badalona, Spain) with a Chemiluminescence Immunoassay (CLIA) method using a Liaison instrument (DiaSorin Liaison, Stillwater, MN). 25(OH)D is an indirect method to measure vitamin D in the blood. According to the literature, serum 25(OH)D levels equal or above 20 ng/ml were considered normal levels of vitamin D (Michael F. Holick, 2007).To avoid variations during blood sampling, the blood was almost always collected on the same day of the week at approximately the same hour (13-15h). Seasonal fluctuations did not affect sampling because the weather seasons in Haiti are barely defined. *T. Canis* Ig-G antibodies were detected at the IGTP laboratory using a commercial ELISA kit (Ridascreen, R-Biopharm AG, Germany). Results with a sample index above 1.1 were considered positive according to the manufacturer's instructions.

### 1.4.4 Detection of soil-transmitted parasites infections.

Stool samples were collected to detect intestinal parasites in all the participants. Stool samples were processed on the same day of collection at the laboratory of the paediatric hospital of Saint Damien using the Kato-Katz and formalin-gasoline technique (a modification of the formalin-ether sedimentation technique) as described previously (Ahmadi and Damraj, 2009; Katz, N. Chaves, A. Pellegrino, 1972) and were examined with optical microscopy.

### 1.4.5 Cytokine measurement.

Frozen supernatants remaining from the QFT-GIT tubes were used for the measurement of cytokine concentrations using a bead-based multiplex assay (Luminex 11-plex cytokine kit, R&D Systems, UK) and measured by Bioplex manager software (version 5.0, Bio-Rad, USA) according to the manufacturer's instructions. After optimisation experiments, granulocyte-macrophage colony-stimulating factor (GM-CSF), interferon (IFN)-gamma (γ), interleukin (IL)-2, IL-5, IL-10, IL-13, IL-22, IL-17, and tumor necrosis factor (TNF)-alpha (α) were analysed in a 1:8-fold dilution, while IL-1RA and induced protein (IP)-10 were analysed in 1:8, 1:80, and 1:160-fold dilutions. In this way, values were within the detection limits marked in the standard curve. IL-32 and vascular endothelial growth factor (VEGF) were measured by DuoSet ELISA (R&D Systems, UK) because of incompatibilities with the human custom multiplex cytokine kit. ELISA was performed according to the manufacturer's instructions.

### 1.5 Statistical analysis.

Qualitative variables were described using frequencies and percentages. Qualitative variables were described using median and interquartile ranges (IQR), or, using mean and standard deviation (SD) in case the of variables were with a normally distribution, using mean and standard deviation. The independent variables analysed were: sex, age, weight by age, BCG, haemoglobin levels, 25(OH)D levels, ferritin levels, the presence of intestinal helminths, IgG *T. Canis* antibodies, and cytokines responses. The concentration of released cytokines released in response to *M. tuberculosis* antigens (Ag-TB) and phytohemagglutinin (PHA) was calculated by subtracting the concentration measured in the nil tube (Ag-TB, antigen minus nil; and PHA, mitogen minus nil). For all the variables with a normal distribution, the comparison between several groups was performed using the analysis of variance or Student's t-student test (only two groups). However, for variables non-normally distributed variables, the comparison between groups was performed using Kruskal-Wallis and Mann-Whitney tests. In the case of qualitative variables, comparisons were performed using the Fisher's exact test or chi-squared test. The Tukey method (normal distribution) or Benjamini & Hochberg method (non-normal distribution) was used to correct p-values in multiple comparisons.

Multivariate logistic regression was performed to detect variables able to classify individuals among the three different study groups (active TB, LTBI, and uninfected). Participants with a positive QFT-GIT result (TB and LTBI) were analysed separately (Binomial) from those with a negative QFT-GIT result (TB, LTBI, and uninfected; Multinomial). The results were expressed using the odds ratios (OR) and their confidence intervals. Sensitivity and specificity were calculated for assessing the value of performing a diagnostic test. The receiving operating characteristic (ROC) curve was used to evaluate diagnostic accuracy. The optimal cut-off value was identified to maximise the difference between true positive and false positives subjects. The level of significance was set at 0.05.

These analyses were performed using the statistical software IBM SPSS Statistics v. 25 (SPSS, Chicago, US), R package v.3.0.5 (R Foundation for Statistical Computing, Vienna, Austria), and GraphPad PRISM v. 5(GraphPad Software, Inc., San Diego, USA).

### Example 2. Results.

### 2.1. Study subjects.

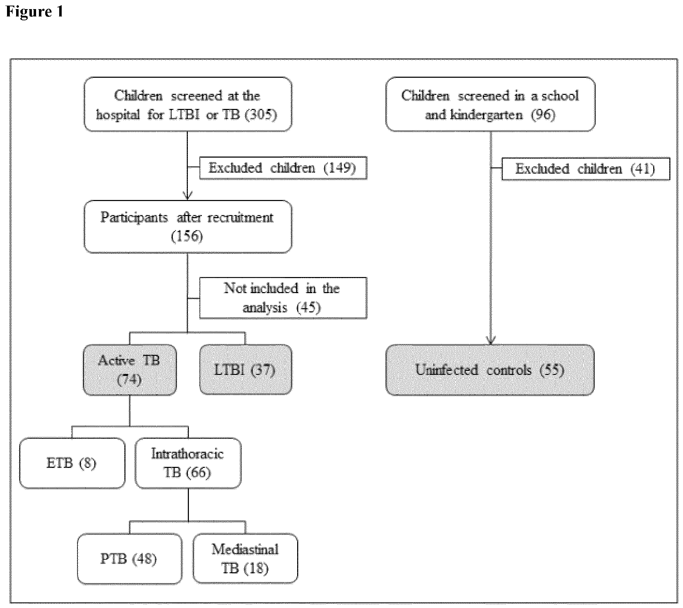

A total of 305 children suspected of having LTBI or TB were screened in the hospital, whereas a total of 96 uninfected children were screened in a school and a kindergarten. Among the patients screened at the hospital, 156 were recruited, and 149 were excluded because they did not meet the inclusion criteria (informed consent not obtained, negative TST). Of the 156 participants, 111 (71.2%) were analysed in the study, and 45 (28,8%) were lost to follow-up and were therefore not included in the analysis due to lack of complementary tests necessary for clinical assessment (chest radiographs, biopsies). Among the children screened at school and kindergarten, 55 were recruited and analysed, and 41were excluded because they did not meet the inclusion criteria (informed consent not obtained, positive TST). Among the 166 children analysed (111 screened at the hospital and 55 screened at school and kindergarten), 74 had active TB (44.6%), 37 had LTBI (22.3%), and 55 were uninfected (33.1%). Of the 74 active TB cases, 8 had ETB, and 66 had intrathoracic TB. Among the intrathoracic TB children, 48 had PTB, and 18 had Mediastinal TB (Figure 1).

**Table 3** shows the description and demographic and clinical characteristics among study groups according to their QFT-GIT result. Significant differences were observed in age and body weight-for-age z-scores (p=0.001 and p=0.001, respectively) among the study groups with different QFT-GIT results (positive, negative, and indeterminate). Whereas LTBI cases with positive QFT-GIT were older than active TB cases with negative QFT-GIT (p=0.002), active TB cases with positive QFT-GIT had lower weight-for-age z-scores than uninfected controls (p=0.001). However, comparisons among QFT-GIT results within each study group (TB, LTBI, and non-infected) were performed,and no significant differences were observed.

**Table 3. Description and comparisons of the demographic and clinical characteristics among study groups according to their QFT-GIT result.**

| **Study groups classified by QFT-GIT result (n=166)** | | | | | | | | |
|---|---|---|---|---|---|---|---|---|
| | **Active TB (n=74)** | | | **LTBI (n=37)** | | | **Uninfected (n=55)** | **p. overall** |
| | **Positive** | **Negative** | **Indeterminate** | **Positive** | **Negative** | **Indeterminate** | **Negative** | |
| | *n*=*62* | *n*=*9* | *n=3* | *n*=*22* | *n*=*12* | *n=3* | *n=55* | |
| **Gender** | | | | | | | | 0.548 |
| Female | 26 (41.9%) | 5 (55.6%) | 2 (66.7%) | 12 (54.5%) | 6 (50.0%) | 3 (100%) | 25 (45.5%) | |
| Male | 36 (58.1%) | 4 (44.4%) | 1 (33.3%) | 10 (45.5%) | 6 (50.0%) | 0 (0%) | 30 (54.5%) | |
| **Age in years** | 85.3 (44.8) | 47.3 (51.6) | 59.0 (32.0)^{a} | 114 (45.6)^{a} | 80.6 (44.6) | 48.7 (19.5) | 76.5 (35.2) | **0.001** |
| Range | | | | | | | | **0.008** |
| ≤ 5 years | 25 (40.3%) | 7(77.8%) | 2(66.7%) | 5 (22.7%) | 6(50.0%) | 3(100%) | 32 (58.2%) | |
| > 5 years | 37 (59.7%) | 2(22.2%) | 1(33.3%) | 17 (77.3%) | 6(50.0%) | 0(0%) | 23 (41.8%) | |
| **Body weight-for-age** | | | | | | | | |
| Z-score (SD) | -1.39 (1.70)^{a} | -1.70 (1.09) | -1.78 (1.06) | -0.66 (0.98) | -1.57 (1.50) | -0.92 (1.23) | -0.30 (1.00) ^{a} | **0.001** |
| **BCG scar** | | | | | | | | 0.424 |
| Yes | 41 (69.5%) | 7 (87.5%) | 2 (66.7%) | 15 (71.4%) | 9 (100%) | 1 (50.0%) | 39 (72.2%) | |
| **Hemoglobin** (g/dl) | 10.4 (2.02) | 9.89 (0.97) | 9.57 (0.84) | 11.2 (1.30) | 11.0 (1.37) | 11.0 (3.33) | 11.5 (0.66) | 0.457 |

| | | | | | | | | |
|---|---|---|---|---|---|---|---|---|
| Categorical variables expressed described using frequencies (n) and percentages (%), and quantitative variables expressed using median and interquartile ranges (IQR) or standard deviation (SD). Statistical differences between pairwise comparisons (a). QFT-GIT, QuantiFERON-TB Gold In-Tube; TB, tuberculosis; LTBI, latent TB infection; Z-score, default classification system used to present child nutritional status; BCG, Bacillus Calmette-Guérin. | | | | | | | | |

### 2.2. Characterization of subjects with a positive QFT-GIT test.

Comparisons between the groups with active TB and positive QFT-GIT were performed (**Table 4**). The Ag-TB levels of IL-17A, GM-CSF, together with the PHA levels of TNF-α, GM-CSF and, IL-13, and median haemoglobin levels showed significant differences between active TB groups (p=0.009, p=0.027, p=0.027, p=0.040 and p=0.042, p=0.006, respectively). Furthermore, pairwise comparisons showed that Ag-TB levels of IL-17A and GM-CSF, together with the PHA levels of TNF-α and IL-13, were higher in children with Mediastinal TB than in those with PTB (p=0.009 and 0.042, p=0.025 and p=0.050, respectively). Likewise, haemoglobin levels were significantly higher in children with Mediastinal TB than in children with PTB or ETB (p=0.014 and p= 0.014, respectively). No biomarker showed significant differences between the PTB and ETB groups. Therefore, the groups of children with PTB and ETB were merged and analysed as a single group (TB, n=44), while children with Mediastinal TB (n=18) were analysed separately.

**Table 4. Description and comparisons of demographic, clinical, and cytokine responses among active TB cases with positive QFT-GIT.**

| **Study subjects with Active TB and a positive QFT-GIT assay classified by disease location (n=62)** | | | | |
|---|---|---|---|---|
| | **PTB** | **ETB** | **Mediastinal TB** *n=18* | **p. overall** |
| | *n=36* | *n=8* | | |
| **Gender** | | | | 0.935 |
| Male | 20 (55.6%) | 5 (62.5%) | 11 (61.1%) | |
| **Age in years** | 76.0 [34.5;115] | 89.5 [55.5;100] | 99.0 [70.2;130] | 0.187 |
| Range | | | | 0.406 |
| < 5 years | 17 (47.2%) | 3 (37.5%) | 5 (27.8%) | |
| > 5 years | 19 (52.8%) | 5 (62.5%) | 13 (72.2%) | |
| **Body weight-for-age** | | | | |
| Z-score | -1.24 [-2.74;0.08] | -2.07 [-2.79; -1.13] | -0.98 [-2.37; -0.21] | 0.334 |
| **BCG scar** | | | | 0.854 |
| Yes | 23 (69.7%) | 5 (62.5%) | 13 (72.2%) | |
| **Hemoglobin** (g/dl) | 10.3 [8.90;11.6] **^{a}** | 9.00 [8.15;10.9] **^{b}** | 12.0 [10.8;12.1] **^{ab}** | **0.006** |
| **Intestinal parasites** | | | | |
| Protozoa (cyst) | 0(0%) | 0(0%) | 2 (33.3%) | 0.308 |
| Helminths (ova) | 0(0%) | 1 (33.3%) | 0 (0%) | 0.120 |
| **Serology *Toxocara spp.*** | | | | 0.099 |
| Negative | 31 (86.1%) | 5 (62.5%) | 11 (64.7%) | |
| Positive | 5 (13.9%) | 3 (37.5%) | 6 (35.3%) | |
| **Ferritin** (ng/ml) | 98.4 [33.0;201] | 138 [78.5;218] | 64.4 [47.4;117] | 0.276 |
| **25 (OH) D** (ng/ml) | 26.6 [20.6;34.5] | 30.2 [26.3;34.2] | 28.6 [21.8;31.2] | 0.735 |
| Range | | | | 1.000 |
| Normal levels | 27 (75.0%) | 6 (75.0%) | 14 (77.8%) | |
| Deficiency | 9 (25.0%) | 2 (25.0%) | 4 (22.2%) | |
| **Cytokine responses** (pg/ml) | | | | |

| Ag-TB | | | | |
|---|---|---|---|---|
| TNF-α | 105 [-46.08;399] | 53.1 [36.3;248] | 136 [66.2;386] | 0.400 |
| IP-10 | 31153 [12825;47686] | 37425 [10304;57206] | 33127 [29369;48717] | 0.778 |
| IL-10 | 39.5 [23.9;59.3] | 36.4 [16.9;56.7] | 52.4 [47.2;62.0] | 0.060 |
| IFN-γ | 3922 [1150;10449] | 3855 [2957;6969] | 7020 [4866;13847] | 0.200 |
| IL-1RA | 27796 [12141;42882] | 25015 [13848;66213] | 23673 [15354;51940] | 0.928 |
| IL-17A | 7.58 [1.51;12.6] **^{a}** | 5.67 [0.00;19.2] | 23.1 [9.13;35.9] **^{a}** | **0.009** |
| GM-CSF | 41.4 [8.04;97.1] **^{a}** | 28.9 [11.5;54.8] **^{b}** | 83.6 [39.6;201] **^{a b}** | **0.027** |
| IL-13 | 716 [315;1350] | 1342 [534;1876] | 1326 [984;1755] | 0.071 |
| IL-5 | 3.32 [0.00;7.05] | 0.00 [0.00;13.5] | 8.59 [3.53;25.7] | 0.204 |
| IL-32 | 30.1 [-122.86;64.1] | 59.4 [-25.44;396] | 77.1 [-10.03;360] | 0.222 |
| VEGF | -381.77 [-784.77; - 120.49] | -903.69 [-1128.04; - 499.36] | -468.37 [-931.27;-173.86] | 0.150 |

| PHA | | | | |
|---|---|---|---|---|
| TNF-α | 4961 [2833;8837] **^{a}** | 4850 [3446;7646] | 9762 [4829;12489] **^{a}** | **0.027** |
| IP-10 | 4718 [2862;7972] | 5335 [2623;16865] | 6952 [814;19877] | 0.840 |
| IL-10 | -2.79 [-11.05;0.00] | 0.00 [-0.30;0.91] | 0.00 [-1.99;0.92] | 0.071 |
| IFN-γ | 1102 [632;3913] | 1029 [876;1878] | 1828 [1131;8723] | 0.272 |
| IL-1RA | 46679 [27310;80379] | 39813 [17196;46881] | 82727 [29720;142991] | 0.311 |
| IL-17A | 39.8 [13.4;129] | 9.07 [6.54;13.1] | 19.8 [8.22;81.8] | 0.057 |
| GM-CSF | 65.2 [34.1;118] | 26.7 [9.59;46.5] **^{b}** | 74.7 [54.8;112] **^{b}** | **0.040** |
| IL-13 | 1211 [664;1970] **^{a}** | 1536 [1132;2023] | 2150 [1423;2608] **^{a}** | **0.042** |
| IL-5 | 4.28 [0.00;7.38] | 0.00 [0.00; 0.00] | 2.98 [0.52;4.76] | 0.064 |
| IL-32 | 43.9 [-74.11;243] | 7.99 [-98.12;686] | 243 [69.0;739] | 0.109 |
| VEGF | -62.75 [-368.65;228] | -434.83 [-559.52; -156.10] | -73.02 [-503.76;517] | 0.297 |

| Nil | | | | |
|---|---|---|---|---|
| TNF-α | 151 [40.3;250] | 33.6 [17.3;178] | 106 [55.3;209] | 0.388 |
| IP-10 | 673 [412;1220] | 745 [529;1082] | 973 [616;1201] | 0.573 |
| IL-10 | 8.52 [1.62;16.3] | 0.88 [0.00;5.10] | 5.45 [3.49;9.86] | 0.168 |
| IFN-γ | 197 [114;509] | 310 [106;454] | 200 [171;451] | 0.872 |
| IL-1RA | 14867 [9160;22252] | 11778 [6783;27489] | 12406 [8385;20155] | 0.765 |
| IL-17A | 0.00 [0.00;11.3] | 0.00 [0.00; 0.00] | 3.33 [0.00;19.7] | 0.239 |
| GM-CSF | 1.92 [0.00;16.2] | 0.00 [0.00;2.41] | 2.67 [0.85;5.27] | 0.414 |
| IL-13 | 311 [147;622] | 257 [0.00;423] | 341 [292;444] | 0.604 |
| IL-5 | 0.00 [0.00;5.88] | 0.00 [0.00;8.95] | 3.26 [0.00;5.52] | 0.937 |
| IL-32 | 1096 [508;2241] | 1606 [699;2579] | 1558 [1101;2840] | 0.376 |
| VEGF | 702 [175;1801] | 935 [726;1128] | 693 [368;1323] | 0.830 |

| | | | | |
|---|---|---|---|---|
| Categorical variables expressed described using frequencies (n) and percentages (%), and quantitative variables expressed using median and interquartile ranges (IQR) or standard deviation (SD). Statistical differences between pairwise comparisons (a, b). QFT-GIT, QuantiFERON-TB Gold In-Tube; TB, tuberculosis; PTB, Intrathoracic TB with pulmonary involvement; ETB, Extrathoracic TB; Mediastinal TB, Intrathoracic TB with isolated mediastinal lymphadenopathy in the absence of lung parenchyma involvement; Z-score, default classification system used to present child nutritional status; BCG, Bacillus Calmette-Guérin; 25(OH)D, 25-hydroxyvitamin D; Ag-TB, antigen-dependent response; PHA, mitogen-induced response. | | | | |

Comparisons between the TB group (n=44) and LTBI cases with a positive QFT-GIT (n=22) were performed (**Table 5**). The Ag-TB levels of IP-10 along with ferritin levels were significantly higher in children with TB than in children with LTBI (p=0.005 and p=0.019, respectively). However, the PHA levels of IFN-γ were significantly lower in children with TB than in those with LTBI (p<0.001). Although nil levels of IL-5 showed significant differences between groups (p=0.007), this cytokine was not considered in the analysis because almost all the results of the LTBI cases (20/22) and more than half of those of the children who belonged to the TB group (24/44) were below the standard curve. Regarding the association of *T. canis* with IL-5, no significant differences were found between Ag-TB, PHA and nil IL-5 levels and *T. canis* in the groups with positive QFT-GIT (p-values of 0.202, 0.508, and 0.053, respectively). Moreover, the eosinophil count was determined, but because only 25% of the children had an eosinophil count, this variable was not included in the analysis of the study. Despite the few results, no significant differences were found between Ag-TB, PHA and nil IL-5 levels and eosinophil count in the groups with a positive QFT-GIT (p-values of 0.848, 0.923, and 0.768, respectively).

**Table 5. Description and comparisons of demographic, clinical, and cytokine responses between groups with a positive QFT-GIT.**

| **Subjects with a positive QFT-GIT assay (n=66)** | | | |
|---|---|---|---|
| | **Active TB*** | **LTBI** | **p. overall** |
| | *n=44* | *n=22* | |
| **Body weight-for-age** | | | |
| Z-score | -1.32 [-2.74; -0.17] | -0.64 [-1.58;0.08] | 0.061 |
| **Intestinal parasites** | | | |
| Protozoa (cyst) | 0(0%) | 1 (50.0%) | 0.200 |
| Helminths (ova) | 1 (5.88%) | 1 (16.7%) | 0.462 |
| ***Toxocara spp.*** | | | 0.324 |
| Negative | 36 (81.8%) | 13 (68.4%) | |
| Positive | 8 (18.2%) | 6(31.6%) | |
| **Ferritin** (ng/ml) | 109 [38.2;201] | 52.2 [32.5;70.9] | **0.019** |
| **25 (OH) D** (ng/ml) | 27.6 [20.8;34.5] | 24.7 [21.2;30.3] | 0.563 |
| Range | | | 0.755 |
| Normal levels | 33 (75.0%) | 18 (81.8%) | |
| Deficiency | 11 (25.0%) | 4 (18.2%) | |
| **Cytokine responses** (pg/ml) | | | |

| Ag-TB | | | |
|---|---|---|---|
| TNF-α | 71.0 [-40.32;364] | 47.5 [-26.19;151] | 0.654 |
| IP-10 | 31545 [12093;48379] | 7762 [3676;26629] | **0.005** |
| IL-10 | 39.3 [22.1;59.3] | 22.9 [12.1;44.2] | 0.121 |
| IFN-γ | 3922 [1201;10427] | 1832 [672;9569] | 0.221 |
| IL-1RA | 27796 [12141;48396] | 13918 [4821;33546] | 0.138 |
| IL-17A | 7.58 [0.40;14.9] | 10.5 [0.88;60.2] | 0.173 |
| GM-CSF | 34.5 [8.04;97.1] | 30.2 [18.4;60.3] | 0.828 |
| IL-13 | 728 [315;1468] | 427 [171;1229] | 0.118 |
| IL-5 | 2.90 [0.00;7.05] | 0.00 [0.00;8.99] | 0.579 |
| IL-32 | 34.7 [-99.73;72.6] | -63.80 [-176.88;59.4] | 0.206 |
| VEGF | -442.15 [-913.46; -138.11] | -357.76 [-1013.21; -81.31] | 0.775 |

| PHA | | | |
|---|---|---|---|
| TNF-α | 4850 [2833;8837] | 6290 [4103;9904] | 0.226 |
| IP-10 | 4718 [2680;9102] | 4392 [1800;8974] | 0.644 |
| IL-10 | -0.47 [-9.25;0.00] | -5.14 [-8.32; -1.05] | 0.307 |
| IFN-γ | 1102 [717;2984] | 4420 [2137;11309] | **<0.001** |
| IL-1RA | 42206 [27310;69517] | 45310 [27013;112972] | 0.812 |
| IL-17A | 26.0 [9.06;112] | 99.5 [18.9;229] | 0.053 |
| GM-CSF | 55.1 [24.4;103] | 79.4 [47.3;135] | 0.121 |
| IL-13 | 1232 [683;1973] | 1180 [796;1812] | 0.946 |
| IL-5 | 2.92 [0.00;6.98] | 0.91 [0.00;10.1] | 0.967 |
| IL-32 | 32.9 [-74.11;304] | -1.83 [-57.38;75.4] | 0.391 |
| VEGF | -108.91 [-462.16;181] | -315.24 [-871.29; -16.37] | 0.135 |

| Nil | | | |
|---|---|---|---|
| TNF-α | 139 [35.0;250] | 90.0 [38.0;185] | 0.654 |
| IP-10 | 680 [423;1189] | 435 [208;1208] | 0.161 |
| IL-10 | 7.63 [0.19;14.9] | 9.40 [5.28;13.0] | 0.526 |
| IFN-γ | 226 [111;508] | 241 [179;388] | 0.523 |
| IL-1RA | 14041 [8927;23001] | 11090 [6497;17128] | 0.106 |
| IL-17A | 0.00 [0.00;10.4] | 0.00 [0.00;2.46] | 0.764 |
| GM-CSF | 0.89 [0.00;12.3] | 0.83 [0.00;5.30] | 0.429 |
| IL-13 | 302 [114;595] | 256 [146;499] | 0.683 |
| IL-5 | 0.00 [0.00;6.96] | 0.00 [0.00;0.00] | **0.007** |
| IL-32 | 1112 [556;2241] | 1726 [1033;2590] | 0.334 |
| VEGF | 809 [209;1610] | 688 [317;1381] | 0.870 |

| | | | |
|---|---|---|---|
| Categorical variables expressed described using frequencies (n) and percentages (%), and quantitative variables expressed using median and interquartile ranges (IQR) or standard deviation (SD). QFT-GIT, QuantiFERON-TB Gold In-Tube; TB, tuberculosis; LTBI, latent TB infection; Z-score, default classification system used to present child nutritional status; BCG, Bacillus Calmette-Guérin; 25(OH)D, 25-hydroxyvitamin D; Ag-TB, antigen-dependent response; PHA, mitogen-induced response. *In this table, children with Mediastinal TB were not included into the active TB cases. | | | |

### 2.2. Characterization of subjects with a negative QFT-GIT test.

Comparisons among active TB cases (n=9), LTBI cases (n=12) and uninfected controls (n=55) with a negative QFT-GIT (n=22) were performed (**Table 6**). The PHA levels of IL-10, IL-13, and IL-32, together with the nil levels of TNF-α and IL-10, the underweight levels, and 25(OH)D levels showed significant differences (p<0.001, p=0.007, and p=0.004, p=0.001 and p=0.044, p=0.001, and p=0.037, respectively). Furthermore, pairwise comparisons showed that PHA levels of IL-10 were significantly higher in TB and LTBI cases than in uninfected controls (p=0.020 and p=0.001, respectively), whereas the PHA levels of IL-32 were significantly higher in LTBI cases than in TB cases, and uninfected controls (p=0.034 and p=0.004, respectively), and the PHA levels of IL-13 were significantly higher in LTBI cases compared to uninfected controls (p=0.006). Otherwise, the nil TNF-α levels were significantly higher in uninfected controls compared to LTBI cases (p<0.001). Regarding levels of malnourishment, the weight-for-age z-scores were significantly lower in TB and LTBI cases than in uninfected controls (p=0.006 and p=0.006, respectively). On the contrary, the median 25(OH)D levels were significantly higher in LTBI cases compared to uninfected controls (p=0.022).

**Table 6. Description and comparisons of demographic, clinical, and cytokine responses between groups with a negative QFT-GIT.**

| **Subjects with a Negative QFT-GIT assay (n=76)** | | | | |
|---|---|---|---|---|
| | **Active TB** | **LTBI** | **Uninfected** | **p. overall** |
| | *n=9* | *n=12* | *n=55* | |
| **Body weight-for-age** | | | | |
| Z-score (SD) | -1.44 [-2.45; -1.00] **^{a}** | -1.55 [-2.03; -0.58] **^{b}** | -0.59 [-1.08;0.32] **^{a b}** | **0.001** |
| **Intestinal parasites** | | | | |
| Protozoa (cyst) | 0 (0%) | 3 (50.0%) | 0(0%) | 0.464 |
| Helminths (ova) | 0(0%) | 0(0%) | 2 (4.55%) | 1.000 |
| ***Toxocara spp.*** | | | | 0.314 |
| Negative | 6 (66.7%) | 7 (58.3%) | 42 (77.8%) | |
| Positive | 3 (33.3%) | 5 (41.7%) | 12 (22.2%) | |
| **Ferritin** (ng/ml) | 68.5 [51.0;84.6] | 44.0 [28.4;77.7] | 37.1 [20.3;55.4] | 0.075 |
| **25 (OH) D** (ng/ml) | 29.6 [24.1;30.1] | 30.4 [27.7;34.4] **^{b}** | 26.5 [24.6;30.0] **^{b}** | **0.037** |
| Range | | | | |
| Deficiency | 0(0%) | 0(0%) | 2 (3.84%) | |
| **Cytokine response** (pg/ml) | | | | |

| Ag-TB | | | | |
|---|---|---|---|---|
| TNF-α | -26.62 [-59.20;4.27] | -7.89 [-37.82;0.51] | -57.31 [-321.36;72.9] | 0.385 |
| IP-10 | 544 [-34.59;795] | 94.6 [49.9;1332] | 219 [85.6;594] | 0.894 |
| IL-10 | 1.44 [-2.71;9.21] | 3.77 [0.85;6.40] | 0.85 [-1.64;9.29] | 0.918 |
| IFN-γ | 88.4 [0.00;196] | 54.4 [-5.32;239] | 23.3 [-2.05;82.1] | 0.525 |
| IL-1RA | 4361 [1455;9833] | 3204 [1280;7608] | 3812 [1853;9435] | 0.886 |
| IL-17A | 0.00 [0.00;0.00] | 0.00 [-22.20;6.61] | 0.00 [0.00;1.12] | 0.751 |
| GM-CSF | 1.45 [-1.10;5.08] | 0.00 [0.00;1.45] | 0.04 [-5.15;1.52] | 0.670 |
| IL-13 | 361 [67.3 ;591] | 81.5 [23.5;565] | 56.0 [0.00;200] | 0.240 |
| IL-5 | 0.00 [-0.46;0.00] | 0.00 [0.00;0.49] | 0.00 [0.00;1.88] | 0.171 |
| IL-32 | 5.89 [-145.98;222] | 106 [-143.37;256] | -52.81 [-218.53; -6.17] | 0.197 |
| VEGF | -769.46 [-1673.90;0.00] | -57.79 [-1369.04;19.9] | -243.45 [-466.44; -22.59] | 0.295 |

| PHA | | | | |
|---|---|---|---|---|
| TNF-α | 7244 [2691;8401] | 4609 [2837;6360] | 6932 [5104;9965] | 0.261 |
| IP-10 | 7177 [4600;11211] | 6415 [5166;9139] | 6524 [3733;10553] | 0.819 |
| IL-10 | -8.92 [-13.01; -4.87] **^{a}** | -9.43 [-11.82; -7.98] **^{b}** | -0.43 [-6.29;0.00] **^{a b}** | **<0.001** |
| IFN-γ | 3092 [2179;4970] | 4808 [3212;9358] | 3998 [1897;7064] | 0.536 |
| IL-1RA | 72506 [42906;111675] | 148877 [70665;201214] | 68339 [30968;172755] | 0.184 |
| IL-17A | 105 [8.75;180] | 131 [80.7;224] | 80.0 [30.4;216] | 0.390 |
| GM-CSF | 39.3 [33.8;99.2] | 64.1 [46.6;93.7] | 71.5 [42.6;143] | 0.186 |
| IL-13 | 1948 [1467;2146] | 2400 [2021;2978] **^{b}** | 1563 [789;2236] **^{b}** | **0.007** |
| IL-5 | 5.24 [0.91;5.69] | 2.99 [0.00;11.4] | 3.42 [1.15;6.68] | 0.789 |
| IL-32 | -198.07 [-315.19; -9.88] **^{c}** | 280 [67.2;367] **^{b c}** | -24.34 [-160.79;66.4] **^{b}** | **0.004** |
| VEGF | 0.00 [-853.06;782] | 0.00 [-780.01;374] | -27.36 [-358.31;138] | 0.703 |

| Nil | | | | |
|---|---|---|---|---|
| TNF-α | 228 [71.7;300] | 69.5 [40.4;99.2] **^{b}** | 312 [155;809] **^{b}** | **0.001** |
| IP-10 | 938 [301;1667] | 561 [413;2085] | 487 [317;770] | 0.291 |
| IL-10 | 12.3 [8.92;27.5] | 14.0 [10.1;16.5] | 7.75 [3.59;16.0] | **0.044** |
| IFN-γ | 321 [0.00;862] | 255 [67.9;440] | 199 [89.0;434] | 0.911 |
| IL-1RA | 11598 [7467;18694] | 10372 [8682;16165] | 13409 [9432;22543] | 0.304 |
| IL-17A | 0.00 [0.00;44.3] | 0.00 [0.00;26.5] | 0.00 [0.00;0.71] | 0.220 |
| GM-CSF | 4.98 [0.00;9.43] | 0.00 [0.00;3.76] | 1.30 [0.00;14.6] | 0.258 |
| IL-13 | 1166 [478;1519] | 376 [324;727] | 373 [206;947] | 0.069 |
| IL-5 | 0.46 [0.00;4.96] | 0.00 [0.00;2.49] | 3.07 [0.00;5.52] | 0.122 |
| IL-32 | 1242 [1006;1740] | 2020 [1384;4215] | 1532 [657;3754] | 0.332 |
| VEGF | 1029 [626;1674] | 1546 [491;2629] | 484 [222;1189] | 0.113 |

| | | | | |
|---|---|---|---|---|
| Categorical variables expressed described using frequencies (n) and percentages (%), and quantitative variables expressed using median and interquartile ranges (IQR) or standard deviation (SD). Statistical differences between pairwise comparisons corrections (a, b, c). QFT-GIT, QuantiFERON-TB Gold In-Tube; TB, tuberculosis; LTBI, latent TB infection; Z-score, default classification system used to present child nutritional status; BCG, Bacillus Calmette-Guérin; 25(OH)D, 25-hydroxyvitamin D; Ag-TB, antigen-dependent response; PHA, mitogen-induced response. | | | | |

### 2.3. Discriminative biomarker profiles in subjects with positive QFT-GIT.

The adjusted logistic model showed a set of 4 biomarkers able to discriminate between active TB and LTBI: ferritin, 25(OH)D, IP-10, and IFN-γ (**Table 1**).

**Table 1. Percentages of classification (%) and area under the ROC curve of the study groups.**

| **Model** | **Signature: active TB* vs LTBI** | **Patient classification** | | **AUC** |
|---|---|---|---|---|
| | | **% TB** | **% LTBI** | |
| **1** | **Ag** (IFN-γ) | 100 | 0 | 0.59 |
| **2** | **Ag** (IFN-γ + IP-10) | 88.6 | 27.3 | 0.72 |
| **3** | **Ag** (IFN-γ + IP-10) + **Mit** (IFN-γ + IP-10) | 86.4 | 59.1 | 0.84 |
| **4** | **Ag** (IFN-γ + IP-10) + **Mit** (IFN-γ + IP-10)+ Ferritin | 90.9 | 70.0 | 0.91 |
| **5** | **Ag** (IFN-γ + IP-10) + **Mit** (IFN-γ + IP-10)+ 25(OH)D | 90.9 | 63.6 | 0.87 |
| **6** | **Ag** (IFN-γ + IP-10) + **Mit** (IFN-γ + IP-10)+Ferritin + 25(OH) D | 90.0 | 93.2 | 0.95 |

| | | | | |
|---|---|---|---|---|
| TB, tuberculosis; LTBI, latent TB infection; 25(OH)D, 25-hydroxyvitamin D; **Ag,** lymphocytes stimulated with Ag-TB (QFT). **Mit,** lymphocytes stimulated with mitogen (PHA). *In this table, patients with mediastinal TB were not included into the active TB cases since they are analyzed separately in **Table 2** | | | | |

The best model included 6 variables. The coefficients of this model were showed in **Table 7.** For each increased unit of IFN-γ (in Ag-TB responses), IP-10 (in Ag-TB and PHA responses), ferritin, and 25(OH)D, the odds of being classified as TB case will increase by a factor of 1.08, 1.80, 1.13, 1.02, and 1.22, respectively. However, for each increased unit of IFN-γ (in PHA responses), the odds of being classified as a TB case will decrease by a factor of 0.46. Therefore, the profile to classify a subject with TB instead of LTBI will be that with low levels of IFN-γ (in PHA responses) but high levels of IP-10 (in Ag-TB and PHA responses), 25(OH)D, and ferritin.

**Table 7. Classification table of the model for the study groups with positive QFT-GIT results and variables in the equation.**

| **Classification table of the Active TB* and LTBI cases with positive QFT-GIT results and variables in the equation** | | | |
|---|---|---|---|
| | **B** | **P-value** | **Odds Ratio [95% CI]** |
| **25 (OH) D (ng/ml)** | 0.20 | **0.012** | 1.22 [1.05; 1.43] |
| **Ferritin** (ng/ml) | 0.02 | **0.010** | 1.02 [1.01; 1.03] |

| Ag-TB (pg/ml) ^{†} | | | |
|---|---|---|---|
| **IP-10** | 0.08 | **0.017** | 1.08 [1.01; 1.15] |
| **INF-γ** | 0.12 | 0.064 | 1.13 [0.99; 1.29] |

| PHA (pg/ml) ^{†} | | | |
|---|---|---|---|
| **IP-10** | 0.59 | **0.007** | 1.80 [1.18; 2.75] |
| **INF-γ** | -0.77 | **0.008** | 0.46 [0.26; 0.82] |
| *Constant* | -8.67 | 0.004 | |

| | | | |
|---|---|---|---|
| Binomial Logistic Regression. B, regression coefficient; OR, Odds Ratio; CI, Confidence Interval. QFT-GIT, QuantiFERON-TB Gold In-Tube; TB, tuberculosis; LTBI, LTBI; 25(OH)D, 25-hydroxyvitamin D; Ag-TB, antigen-dependent response; PHA, mitogen-induced response. *In this table, children with Mediastinal TB were not included into the active TB cases. ^{†}Cytokine levels were multiplied x10. | | | |

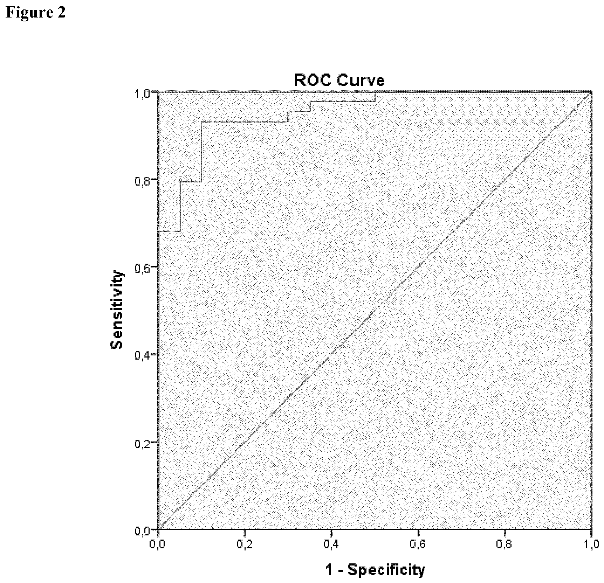

The model based on the four host-markers mentioned above was able to correctly classify 93.2% of children with TB, and 90.0% of children with LTBI. In addition, the area under the ROC curve of this model was 0.955 (CI 95%: 0.91 to 1.00), and the positive and negative likelihood ratio were 9.32 and 0.08, respectively (Figure 2). Furthermore, when this model was applied to Mediastinal TB (i.e. early stages of TB) to compare with LTBI, the model classified correctly 76.5%, and the area under the ROC curve was 0.83 (CI95%;0.7 to 0.96).

**Table 2. Percentages of classification (%) and area under the ROC curve of the study groups.**

| **Model** | **Signature: Mediastinal TB vs LTBI** | **Patient classification** | | **AUC** |
|---|---|---|---|---|
| | | **% Early stages of TB** | **% LTBI** | |
| **1** | **Ag** (IFN-γ) | 100% | 0.0% | 0.5 |
| **2** | **Ag** (IFN-γ + IP-10) | 88.9% | 27.3% | 0.58 |
| **3** | **Ag** (IFN-γ + IP-10) + **Mit** (IFN-γ + IP-10) | 83.3% | 59.1% | 0.71 |
| **4** | **Ag** (IFN-γ + IP-10) + **Mit** (IFN-γ + IP-10)+ Ferritin | 88.2% | 70.0% | 0.79 |
| **5** | **Ag** (IFN-γ + IP-10) + **Mit** (IFN-γ + IP-10)+ 25(OH)D | 88.9% | 63.6% | 0.76 |
| **6** | **Ag** (IFN-γ + IP-10) + **Mit** (IFN-γ + IP-10)+Ferritin + 25(OH) D | 76.5% | 93.2% | 0.83 |

| | | | | |
|---|---|---|---|---|
| TB, tuberculosis; **Ag,** lymphocytes stimulated with Ag-TB (QFT). **Mit,** lymphocytes stimulated with mitogen (PHA); 25(OH)D, 25-hydroxyvitamin D. | | | | |

### 2.4.Discriminative biomarker profiles in subjects with negative QFT-GIT.

The adjusted logistic model showed a set of 3 biomarkers, namely, IL-10, IL-13, and IL-32, able to discriminate among active TB, LTBI, and uninfected controls (**Table 8**). For each increased unit of IL-13 and IL-32 (in PHA responses), the odds of being classified as LTBI case will increase by a factor of 1.003 and 1.003, respectively. However, for each increased unit of IL-10 (in PHA responses), the odds of being classified as uninfected control will decrease by a factor of 0.93 and 0.01, respectively.

The combination of PHA levels of IL-10, IL-13, and IL-32 detected 11.1% of active TB cases, 50.0% of LTBI cases, and 96.4% of uninfected controls.

**Table 8. Classification table for the study groups with negative QFT-GIT results and variables in the equation.**

| **Classification table of the study groups with negative QFT-GIT results and variables in the equation** | | | |
|---|---|---|---|
| **Model ^{a}** | **B** | **P-value** | **Odds Ratio [95% CI]** |
| **Active TB** | | | |
| PHA (pg/ml) | | | |
| *Constant* | -4.67 | | |
| **IL-10** | -0.071 | **0.036** | 0.93 [0.87; 0.99] |
| **IL-13** | 1.25x10⁻³ | 0.073 | 1.01 [1.00; 1.01] |
| **IL-32** | 3.42x10⁻⁴ | 0.751 | 1.00 [0.99. 1.01] |

| **LTBI** | | | |
|---|---|---|---|
| PHA (pg/ml) | | | |
| *Constant* | -8.873 | | |
| **IL-10** | -0.095 | **0.009** | 0.910 [0.85; 0.98] |
| **IL-13** | 3.06x10⁻³ | **0.001** | 1.003 [1.001; 1.005] |
| **IL-32** | 2.60x10⁻³ | **0.007** | 1.003 [1.001; 1.005] |

| | | | |
|---|---|---|---|
| ^{a} The reference category is the uninfected control group. Multivariate Logistic Regression. B, regression coefficient; OR, Odds Ratio; CI, Confidence Interval. QFT-GIT, QuantiFERON-TB Gold In-Tube; TB, tuberculosis; LTBI, LTBI; PHA, mitogen-induced response. | | | |

## Claims

1. I*n vitro* method for the differential diagnosis between active TB and LTBI which comprises:
a. Determining the level of at least IFN-γ and IP-10 after the stimulation of blood samples obtained from the patient with a *M. tuberculosis* antigen;
b. Determining the level of at least IFN-γ and IP-10 after the stimulation of blood samples obtained from the patient with a mitogen;
c. Wherein if the level of IFN-γ after the stimulation with the mitogen is lower as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is an indication that the patient is suffering from active TB;
d. Wherein if the level of IFN-γ after the stimulation with a mitogen is higher as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is an indication that the patient is suffering from LTBI.

2. *In vitro* method, according to the claim 1, wherein if the level of IFN-γ after the stimulation with the mitogen is lower as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is also an indication that the patient is suffering from early stage active TB.

3. *In vitro* method, according to any of the claims 1 or 2, further comprising the determination of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin, and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol, wherein if the level of the acute-phase reactant and/or the level of the vitamin D analogue is higher as compared with the level obtained in patients suffering from LTBI, this is an indication of active TB, preferably early stage active TB.

4. *In vitro* method for predicting the response to a treatment of patients suffering from active TB which comprises:
a. Determining the level of at least IFN-γ and IP-10 after the stimulation of blood samples obtained from the patient with a *M. tuberculosis* antigen;
b. Determining the level of at least IFN-γ and IP-10 after the stimulation of blood samples obtained from the patient with a mitogen;
c. Wherein if the level of IFN-γ after the stimulation with a mitogen is higher as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, this is an indication that the patient is responding to the treatment.

5. *In vitro* method, according to claim 4, further comprising the determination of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol wherein if the level of the acute-phase reactant and/or vitamin D analogue is lower as compared with the level obtained in patients suffering from active TB, this is an indication that the patient is responding to the treatment.

6. *In vitro* method for deciding whether to recommend a treatment to patients suffering from active TB which comprises:
a. Determining the level of at least IFN-γ and IP-10 after the stimulation of blood samples obtained from the patient with a *M. tuberculosis* antigen;
b. Determining the level of at least IFN-γ and IP-10 after the stimulation of blood samples obtained from the patient with a mitogen;
c. Wherein if the level of IFN-γ after the stimulation with the mitogen is lower as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, an anti-TB treatment is recommended.

7. *In vitro* method for deciding whether to recommend a preventive treatment to patients suffering from LTBI which comprises:
a. Determining the level of at least IFN-γ and IP-10 after the stimulation of blood samples obtained from the patient with a *M. tuberculosis* antigen;
b. Determining the level of at least IFN-γ and IP-10 after the stimulation of blood samples obtained from the patient with a mitogen;
c. Wherein if the level of IFN-γ after the stimulation with a mitogen is higher as compared with the level of IFN-γ obtained after the stimulation with the antigen, and the level of IP-10 and IFN-γ after stimulation with the antigen and the mitogen are higher as compared with the level of IP-10 and IFN-γ obtained without stimulation, a preventive treatment is recommended.

8. *In vitro* method, according to any of the claims 6 or 7, further comprising the determination of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol and/or falecalcitriol wherein if the level of the acute-phase reactant and/or vitamin D analogue is higher as compared with the level obtained in patients suffering from LTBI, an anti-TB treatment or a preventive treatment is recommended.

9. *In vitro* method, according to any of the previous claims, wherein the level of IFN-γ and IP-10 is directly determined by ELISA or indirectly determined by counting the number of T cells secreting IFN-γ and IP-10.

10. *In vitro* method, according to any of the previous claims wherein the mitogen is phytohaemagglutinin and the *M. tuberculosis* antigen are ESAT-6 and/or CFP-10.

11. *In vitro* method, according to any of the previous claims wherein the result is confirmed by CT scan, mycobacterial culture, PCR *of M. tuberculosis,* or by pathological anatomy.

12. *In vitro* use of at least IFN-γ and IP-10, after the stimulation of blood samples obtained from the patient with a *M. tuberculosis* antigen and with a mitogen, for the differential diagnosis between active TB and LTBI, for the diagnosis of early stage active TB, for predicting the response to a treatment of patients suffering from active TB, for deciding whether to recommend a treatment to patients suffering from active TB or for deciding whether to recommend a preventive treatment to patients suffering from LTBI.

13. *In vitro* use, according to claim **11,** in combination with the level of an acute-phase reactant selected from the list comprising: ferritin, c-reactive protein (CRP), procalcitonin (PCT), lactate dehydrogenase (LDH), erythrocyte sedimentation rate (ESR) or lactoferrin and/or the level of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol.

14. Kit adapted for the differential diagnosis between active TB and LTBI, for the diagnosis of early stage active TB, for predicting the response to a treatment of patients suffering from active TB, for deciding whether to recommend a treatment to patients suffering from active TB or for deciding whether to recommend a preventive treatment to patients suffering from LTBI, which comprises:
a. Reagents or media for the determination of the level of IFN-γ and IP-10 after the stimulation of blood samples obtained from the patient with a *M. tuberculosis* antigen, and
b. Reagents or media for the determination of the level of IFN-γ and IP-10 after the stimulation of blood samples obtained from the patient with a mitogen.

15. Kit, according to claim 14, further comprising reagents or media for the detection of the level of an acute-phase reactant selected from the list comprising: ferritin, c-reactive protein (CRP), procalcitonin (PCT), lactate dehydrogenase (LDH), erythrocyte sedimentation rate(ESR) or lactoferrin and/or for the detection of a vitamin D analogue selected from the list comprising: 25-hydroxyvitamin D, paricalcitol, calcitriol, calcidiol, alfacalcidol, tacalcitol, calcipotriol, maxacalcitol, doxercalciferol or falecalcitriol.
